# EUROPÄISCHE PATENTSCHRIFT

(11) **EP 0 423 394 B1**
(45) Veröffentlichungstag und Bekanntmachung des Hinweises auf die Patenterteilung: **09.06.1993**
(21) Anmeldenummer: 89119524.0
(22) Anmeldetag: 20.10.1989
(51) Int. Cl.: G01P 13/00

(54) **Induktiver Bewegungssensor**
Inductive movement sensor
Capteur inductif de mouvement

(43) Veröffentlichungstag der Anmeldung: 24.04.1991
(73) Patentinhaber: Siemens-Elema AB, 171 95 Solna 1 (SE); SIEMENS AKTIENGESELLSCHAFT, 80333 München (DE)
(72) Erfinder: Dalén, Björn, Dipl.Ing., S-113 22 Stockholm (SE); Nilsson, Kenth, Dipl.Ing., S-184 60 Akersberga (SE); Högnelid, Kurt, Dipl.Ing., S-172 35 Sundbyberg (SE); Wecke, Liliane, Dipl.Ing., S-172 38 Sundbyberg (SE)

(56) Entgegenhaltungen:
- EP-A- 0 254 945
- US-A- 4 656 458

## Beschreibung

Die Erfindung betrifft einen mit einem Objekt verbindbaren induktiven Bewegungssensor, welcher eine Spule und einen relativ zu der Spule beweglichen Magnetkörper aufweist.

Ein solcher Bewegungssensor ist aus dem Dokument EP-A-254 945 bekannt. Das Dokument beschreibt u.a. einen induktiven Sensor, der im wesentlichen aus einem Hohlkörper, einem darin frei beweglichen magnetischen Dipol und einer auf dem Hohlkörper angeordneten Spule besteht, wobei der Sensor durch eine geeignete, z.B. ellipsoidische, Form des Hohlkörpers in seiner Empfindlichkeit richtungsabhängig ist.

Die Funktion derartiger Bewegungssensoren beruht darauf, daß bei Bewegungen des Objektes, an dem der Bewegungssensor angebracht ist, Verlagerungen des Magnetkörpers relativ zu der Spule auftreten, so daß in diese eine elektrische Spannung induziert wird. Wird der Bewegungssensor mit einem Lebewesen verbunden, um dessen körperliche Aktivität zu überwachen, wird der induzierte Strom auf seine Amplitude und/oder zeitliche Änderung hin ausgewertet, um Rückschlüsse auf die Stärke und/oder die Art der körperlichen Aktivität des Lebewesens ziehen zu können. Findet der Bewegungssensor dagegen als Diebstahlsensor Verwendung, genügt es in der Regel, das bloße Auftreten einer induzierten Spannung zu detektieren, um erkennen zu können, daß das zu schützende objekt von seinem Platz entfernt werden soll.

Bei bekannten Bewegungssensoren der eingangs genannten Art tritt insbesondere dann, wenn diese eine hohe Empfindlichkeit besitzen, also bereits auf sehr kleine Bewegungen ansprechen, das Problem auf, daß Störungen infolge des Magnetfeldes der Erde oder infolge von von elektrischen Geräten, z.B. Elektromotoren, ausgehenden niederfrequenten Magnetfeldern auftreten. Insbesondere beim Auftreten niederfrequenter Magnetfelder kommt es häufig zu Fehldetektionen, d.h., daß Bewegungen des Magnetkörpers relativ zu der Spule auftreten, ohne daß eine Bewegung des zu überwachenden Objektes vorliegt.

Der Erfindung liegt die Aufgabe zugrunde, einen Bewegungssensor der eingangs genannten Art so auszubilden, daß Funktionsstörungen infolge des Magnetfeldes der Erde und/oder infolge des Auftretens von niederfrequenten Magnetfeldern sicher vermieden sind.

Nach der Erfindung wird diese Aufgabe dadurch gelöst, daß der Magnetkörper wenigstens vier Magnetpole aufweist, wobei die Anzahl der Südpole gleich der der Nordpole ist, und daß die Polstärke der Magnetpole so bemessen ist und die Magnetpole an dem Magnetkörper so plaziert sind, daß sich der Magnetkörper in einem homogenen Magnetfeld bezüglich der durch das Magnetfeld auf die Magnetpole ausgeübten Kräfte in einer indifferenten Gleichgewichtslage befindet. Da es sich bei dem Magnetfeld der Erde ebenso wie bei von elektrischen Geräten ausgehenden niederfrequenten Magnetfeldern, in letzterem Falle wird angenommen, daß sich der Bewegungssensor nicht in unmittelbarer Nähe des elektrischen Gerätes befindet, um homogene Magnetfelder handelt, können diese somit keine Relativbewegungen des Magnetkörpers relativ zu der Spule verursachen. Damit sind Störungen durch die genannten Magnetfelder ausgeschlossen. Die erforderliche Beweglichkeit des Magnetkörpers relativ zu der Spule kann beispielsweise dadurch erreicht werden, daß dieser an einem Faden oder an einer bzw. mehreren Federn nach Art eines Pendels aufgehängt ist.

Eine besonders zweckmäßige Ausführungsform der Erfindung sieht vor, daß der Bewegungssensor ein einen Hohlraum umschließendes Gehäuse aufweist, wobei der Magnetkörper in dem Hohlraum aufgenommen ist, und daß die Spule das Gehäuse umgibt. Hierdurch wird eine verbesserte Handhabbarkeit des Sensors erreicht, die nochmals verbessert werden kann, wenn die Spule auf die Außenseite des Gehäuses, die dann zweckmäßigerweise nach Art eines Wickelkörpers ausgebildet ist, gewickelt ist. Der Magnetkörper kann auf technisch einfache Weise hergestellt werden, wenn die Magnetpole jeweils die gleiche Polstärke besitzen und in gleichen Winkelabständen voneinander derart auf einer Kreislinie angeordnet sind, daß auf einen Nordpol jeweils ein Südpol folgt. Dies läßt sich dann besonders einfach realisieren, wenn der Magnetkörper gemäß einer Variante der Erfindung kugelförmig ausgebildet ist. Dabei kann vorgesehen sein, daß der kugelförmige Magnetkörper auf der Wandung des Hohlraumes abrollt, wodurch die erforderliche Beweglichkeit des Magnetkörpers relativ zu der Spule auf technisch besonders einfache Weise realisiert ist. In diesem Zusammenhang ist es zweckmäßig, den Hohlraum rotationssymmetrisch auszubilden. Bei kugelförmigem Hohlraum wird der Vorteil erreicht, daß der Sensor völlig lageunabhängig arbeitet. Ist dagegen eine lageabhängige Funktion des Bewegungssensors erwünscht, so kann diese realisiert werden, wenn der Hohlraum derart geformt ist, daß bei wenigstens einer Ausrichtung des Bewegungssensors relativ zur Richtung der Schwerkraft ein Abrollen des Magnetkörpers auf der Wandung des Hohlraums zumindest erschwert ist. Dabei kann der Hohlraum derart ausgebildet sein, daß das Abrollen des Magnetkörpers nur erschwert oder sogar völlig ausgeschlossen ist. Nimmt das mit einem solchen Bewegungssensor versehene Objekt eine solche Lage ein, daß ein Abrollen des Magnetkörpers auf der Wandung des Hohlraumes erschwert bzw. völlig ausgeschlossen ist, weist der Bewegungssensor eine verringerte Empfindlichkeit bzw. die Empfindlichkeit Null auf. Erst wenn sich die Lage des Objektes so weit ändert, daß ein ungestörtes Abrollen des Magnetkörpers auf der Wandung des Hohlraumes wieder möglich ist, ist der Bewegungssensor wieder aktiv. Die lageabhängige Funktion des Bewegungssensors läßt sich auf einfache Weise realisieren, wenn der Hohlraum wenigstens annähernd die Form eines Rotationsellipsiods aufweist oder zitronenförmig ausgebildet ist.

Der erfindungsgemäße Bewegungssensor kann mit besonderem Vorteil als Bewegungssensor in einem Herzschrittmacher, dessen Stimulationsfrequenz in Abhängigkeit von der körperlichen Aktivität des den Herzschrittmacher tragenden Patienten gesteuert wird, Verwendung finden, da im Gegensatz zu bisher zu diesem Zweck verwendeten induktiven Bewegungssensoren nicht durch das Magnetfeld der Erde gedämpft ist, so daß auch geringe Bewegungen, wie sie insbesondere im Zusammenhang mit der körperlichen Aktivität älterer Patienten auftreten, gut erfaßt werden können. Von ganz besonderem Vorteil ist es, wenn in einem Herzschrittmacher ein Bewegungssensor Verwendung findet, der derart lageabhängig arbeitet, daß sowohl bei auf dem Bauch als auch bei auf dem Rücken liegenden Patienten ein Abrollen der Kugel auf der Wandung des Hohlraumes erschwert oder völlig ausgeschlossen ist. In diesem Falle ist eine physiologisch unbegründete Steigerung der Stimulationsfrequenz bei im Zustand der körperlichen Ruhe, insbesondere im Zustand des Schlafes befindlichem Patienten ausgeschlossen, da der Bewegungssensor erst dann wieder ausreichend empfindlich ist, wenn sich der Patient erhebt oder zumindest eine sitzende Position einnimmt. Im Gegensatz zu bekannten piezoelektrischen Bewegungssensoren, hier besteht die Gefahr einer physiologisch unbegründeten Steigerung der Stimulationsfrequenz infolge des bei liegendem Patienten auf dem Sensor lastenden Körpergewichtes, sind bei dem erfindungsgemäßen Bewegungssensor physiologisch unbegründete Steigerungen der Stimulationsfrequenz weitgehend ausgeschlossen.

Eine andere bevorzugte Verwendung findet der erfindungsgemäße Bewegungssensor als Bewegungssensor zur Überwachung eines bettlägerigen Patienten, insbesondere in der Intensivmedizin. Auch hier kommt der Vorteil zum Tragen, daß mittels des erfindungsgemäßen Bewegungssensors bereits geringste Bewegungen detektierbar sind.

Eine weitere bevorzugte Verwendung des erfindungsgemäßen Bewegungssensors sieht vor, daß dieser als Diebstahlsensor verwendet wird. Im Gegensatz zu bekannten Sensoren, die mittels eines starken Permanentmagneten funktionsunfähig gemacht werden können, ist dies im Falle des erfindungsgemäßen Bewegungssensors nicht ohne weiteres möglich, da dessen Magnetkörper in dem Magnetfeld des Permanentmagneten eine indifferente Gleichgewichtslage einnimmt. Nur sehr starke Permanentmagnete, die in unmittelbare Nähe des erfindungsgemäßen Bewegungssensors gebracht werden, vermögen dessen Funktion ernsthaft zu stören.

Die Erfindung wird nachfolgend anhand der beigefügten Zeichnungen verdeutlicht. Es zeigen:
- Fig. 1: einen erfindungsgemäßen Bewegungssensor in geschnittener Darstellung,
- Fig. 2: eine Stirnansicht des Bewegungssensors gemäß Fig. 1,
- Fig. 3: ein das Verhalten eines herkömmlichen zweipoligen Magnetkörpers in einem homogenen Magnetfeld veranschaulichendes Schaubild,
- Fig. 4: ein das Verhalten des Magnetkörpers des erfindungsgemäßen Bewegungssensors in einem homogenen Magnetfeld veranschaulichendes Schaubild,
- Fig. 5: einen einen erfindungsgemäßen Bewegungssensor enthaltenden Herzschrittmacher in grob schematischer Darstellung,
- Fig. 6: einen besonderen Betriebszustand des Bewegungssensors nach den Fig. 1 und 2,
- Fig. 7: die Verwendung des erfindungsgemäßen Bewegungssensors zur Überwachung eines bettlägerigen Patienten,
- Fig. 8: die Verwendung des erfindungsgemäßen Bewegungssensors als Diebstahlsensor, und
- Fig. 9: einen weiteren erfindungsgemäßen Bewegungssensor in geschnittener Darstellung.

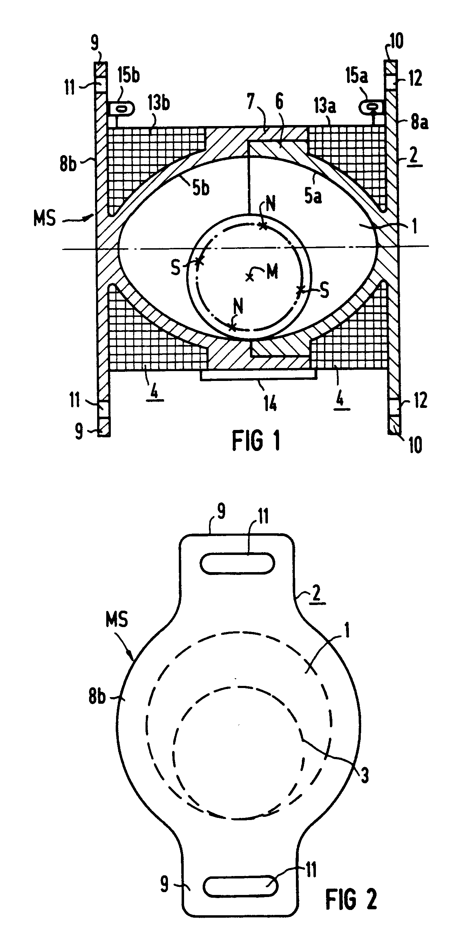

Wie aus der Fig. 1 deutlich wird, weist der erfindungsgemäße Bewegungssensor MS ein einen Hohlraum 1 umschließendes Gehäuse 2, einen innerhalb des Hohlraumes 1 befindlichen Magnetkörper 3 und eine auf der Außenseite des Gehäuses 2 angebrachte Spule 4 auf. Bei Bewegungen des Bewegungssensors MS bzw. eines Objektes, an dem dieser angebracht ist, rollt der Magnetkörper 3 auf der Wandung des Hohlraumes 1 ab. Dabei treten Verlagerungen des Magnetkörpers 3 relativ zu der Spule 4 auf, mit der Folge, daß in letztere eine elektrische Spannung induziert wird, welche das Auftreten einer Bewegung anzeigt.

Das Gehäuse 2 besteht aus zwei Gehäuseteilen 5a und 5b, die in geeigneter Weise, z.B. durch Kleben, miteinander verbunden sind und gemeinsam den Hohlraum 1, der die Gestalt eines Rotationsellipsoids besitzt, begrenzen. Der an den Hohlraum 1 angrenzende Bereich der zwischen den beiden Gehäuseteilen 5a, 5b vorliegenden Trennfuge liegt in einer rechtwinklig zu der strichpunktiert dargestellten Rotationsachse des Rotationsellipsoids verlaufenden Ebene, und zwar an derjenigen Stelle, an der das Rotationsellipsoid seinen größten Durchmesser besitzt. Hierdurch ist erreicht, daß die Gehäuseteile 5a, 5b im Bereich ihrer den Hohlraum 1 begrenzenden Wandungen keine Hinterschneidungen aufweisen, so daß die Gehäuseteile 5a, 5b ohne Probleme als Spritz- oder Druckgußteile hergestellt werden können.

An seinem dem Gehäuseteil 5b zugewandten Ende ist das Gehäuseteil 5a mit einem Zentrieransatz 6 versehen, der eine zylindrische äußere Mantelfläche besitzt, deren Mittelachse der Rotationsachse des Rotationsellipsoids entspricht. Das Gehäuseteil 5b ist an seinem dem Gehäuseteil 5a zugewandten Ende mit einem den Zentrieransatz 6 des Gehäuseteiles 5a aufnehmenden Zentrierrand 7 versehen, der eine zylindrische Innenwand besitzt, deren Durchmesser dem der äußeren Mantelfläche des Zentrieransatzes 6 entspricht. Die Mittelachse Innenwand des Zentrierrandes 7 entspricht ebenfalls der Rotationsachse des Rotationsellipsoids. Die beiden Gehäuseteile 5a, 5b sind also mittels des Zentrieransatzes 6 und des Zentrierrandes 7 derart zueinander zentriert, daß die den Hohlraum 1 begrenzende Wandung des Gehäuses 2 im Bereich der Trennfuge zwischen den Gehäuseteilen 5a, 5b keinerlei Absatz aufweist, wodurch ein ungestörtes Abrollen des Magnetkörpers 3 gewährleistet ist.

Sieht man von dem Zentrieransatz 6 bzw. dem Zentrierrand 7 ab, besitzen die Gehäuseteile 5a, 5b im wesentlichen eine konstante Wandstärke, so daß die äußere Gestalt des Gehäuses 1 im wesentlichen der eines Rotationsellipsoids entspricht. Im Bereich der Spitzen dieses Rotationsellipsoids sind die Gehäuseteile 5a, 5b jeweils mit einem etwa kreisscheibenförmigen Flansch 8a, 8b versehen. Die Flansche 8a, 8b weisen jeweils zwei etwa rechteckige Ansätze 9, 10 auf, die mit Langlöchern 11, 12 versehen sind. Die Flansche 8a, 8b besitzen übrigens jeweils einen Außendurchmesser, der etwa dem Außendurchmesser des Zentrierrandes 7 entspricht.

Die Außenwand des Gehäuseteiles 5a, der Flansch 8a und der Zentrieransatz 6 sowie der Zentrierrand 7 einerseits und die Aussenwand des Gehäuseteiles 5b, der Flansch 8b und der Zentrierrand 7 andererseits begrenzen also zwei Nuten, in die die aus zwei Spulenabschnitten 13a, 13b bestehende Spule 4 gewickelt ist. Die Spule 4 besteht aus Kupferlackdraht mit einer Stärke von 6 bis 100 µm und besitzt je nach Stärke des Kupferlackdrahtes 100 bis 100 000 Windungen. Die beiden Spulenabschnitte 13a, 13b sind an ihren einen Enden mittels einer Leitung 14 derart verbunden, daß ein durch die Spule 4 fließender Strom in beiden Spulenabschnitten 13a, 13b den gleichen Drehsinn besitzt. Die anderen Enden der Spulenabschnitte 13, 13b sind zu schematisch angedeuteten, an den Flanschen 8a, 8b angebrachten Lötstützpunkten 15a, 15b geführt und mit diesen verlötet. An die Lötstützpunkte 15a, 15b können in nicht dargestellter Weise Leitungen angelötet werden, die zur Verbindung der Spule 4 mit einer nicht dargestellten geeigneten Auswerteelektronik dienen.

Die Flansche 8a, 8b mit den Ansätzen 9, 10 dienen übrigens nicht nur zur Fixierung der Spulenabschnitte 13a, 13b, sondern außerdem zu Befestigungszwecken. Im einfachsten Fall kann der Bewegungssensor MS an einem oder beiden Flansche 8a, 8b mit einem zu überwachenden Objekt bzw. mit einem mit diesem fest verbundenen Bauteil oder dergleichen verklebt werden. Es besteht jedoch auch die Möglichkeit, den Bewegungssensor MS mit Hilfe von nicht dargestellten, durch die Langlöcher 11, 12 geführten Schrauben an einem zu überwachenden Objekt bzw. einem mit diesem fest verbundenen Bauteil oder dergleichen zu befestigen. Schließlich kann auch in nicht dargestellter Weise vorgesehen sein, daß der Bewegungssensor eventuell zusammen mit einer Auswerteelektronik in einem Gehäuse aufgenommmen ist, das in geeigneter Weise, z.B. mittels eines Gurtes, an einem zu überwachenden Objekt angebracht wird.

Der in dem Hohlraum 1 aufgenommene Magnetkörper 3 besitzt die Gestalt einer Kugel (Durchmesser etwa 2 mm) und weist vier Magnetpole auf, bei denen es sich um zwei Nordpole N und zwei Südpole S handelt. Die Lage der Magnetpole ist in Fig. 1 durch Kreuze angedeutet. Wie durch einen strichliert eingetragenen Kreis deutlich wird, liegen die Magnetpole auf einer Kreislinie, deren Mittelpunkt auf einer durch den Mittelpunkt M der Kugel verlaufenden Achse liegt. Die Nordpole N und die Südpole S sind einander jeweils diametral gegenüberliegend angeordnet. Außerdem ist die Anordnung derart getroffen, daß zwischen zwei einander benachbarten Polen ein Winkel "Alpha" von 90° liegt.

Weiter ist im Falle des beschriebenen Ausführungsbeispieles vorgesehen, daß die Magnetpole in einer den Mittelpunkt M der Kugel enthaltenden Ebene angeordnet sind. Sämtliche Magnetpole besitzen die gleiche Polstärke, wobei jeder Magnetpol mit den beiden ihm unmittelbar benachbarten Magnetpolen jeweils einen magnetischen Dipol bildet. Infolge dieser Ausbildung des Magnetkörpers 3 befindet sich dieser in einem homogenen Magnetfeld in einem indifferenten Gleichgewichtszustand, was die durch das Magnetfeld auf die Magnetpole ausgeübten Kräfte angeht.

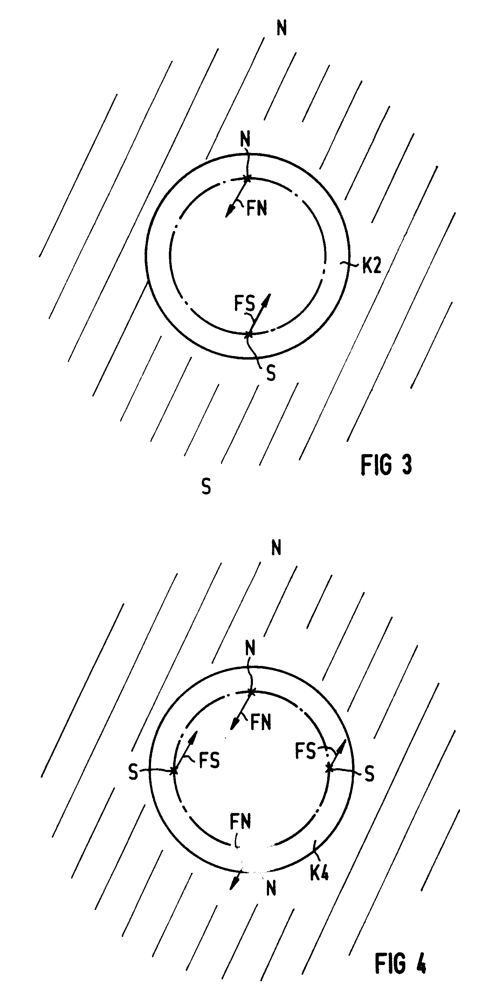

Dies wird anhand der Fig. 3 und 4 ohne weiteres deutlich. Die Fig. 3 zeigt einen kreisscheibenförmigen Magnetkörper MR mit einem Nordpol N und einem diesem diametral gegenüberliegenden Südpol S, der sich in einem homogenen Magnetfeld befindet, dessen Feldlinien durch von Norden N nach Süden S verlaufende Geraden angedeutet sind. Am Nordpol N greift eine Kraft FN an, die parallel zu den Feldlinien in Richtung Süden wirkt und dadurch zustande kommt, daß der Nordpol N das Bestreben hat, sich nach Süden auszurichten. Entsprechend greift an dem Südpol S eine Kraft FS an, die ebenfalls parallel zu den Feldlinien verläuft, jedoch nach Norden gerichtet ist und ihre Ursache darin hat, daß der Südpol S bestrebt ist, sich nach Norden auszurichten. Infolge des Umstandes, daß der Nordpol N und der Südpol S, die einen magneten Dipol bilden, jeweils die gleiche Polstärke besitzen, sind die Kräfte FN und FS dem Betrag nach gleich. Da die beiden Kräfte FN und FS einander exakt entgegengesetzt gerichtet sind, ist die Summe der Kräfte gleich Null. Dies gilt jedoch für die in Fig. 3 dargestellte Lage des Magnetkörpers MR bezüglich der Summe der durch die Kräfte FN und FS ausgeübten Drehmomente offensichtlich nicht. Der Magnetkörper MR wird sich also unter der Wirkung des homogenen Magnetfeldes derart ausrichten, daß der Nordpol N exakt nach Süden und der Südpol exakt nach Norden weist. In dieser Situation ist dann nicht nur die Summe der Kräfte gleich Null, sondern auch die Summe der Drehmomente, da dann die Kraft FN die gleiche Wirkungslinie wie die Kraft FS besitzt. Es wird also deutlich, daß der Magnetkörper MR gemäß Fig. 3 bestrebt ist, eine stabile Gleichgewichtslage einzunehmen, in der sein Nordpol N exakt nach Süden und sein Südpol S exakt nach Norden weist. Überträgt man dies auf einen Bewegungssensor gemäß den Fig. 1 und 2, der anders als im Falle der Erfindung einen kugelförmigen Magnetkörper mit nur zwei Magnetpolen, nämlich einen Nordpol N und einen Südpol S aufweist, wird deutlich, daß ein magnetisches Störfeld eine Lageänderung des Magnetkörpers hervorrufen muß, wodurch in die Spule 4 ein Strom induziert wird, ohne daß das Objekt, mit dem der Bewegungssensor verbunden ist, irgendeine Bewegung ausführt. Außerdem wird der Magnetkörper K2 infolge des Umstandes, daß er sich bezüglich der durch ein homogenes Magnetfeld auf die Magnetspule ausgeübten Kräfte in einem stabilen Gleichgewichtszustand befindet, das stets vorhandene Magnetfeld der Erde bedämpft. Dies hat zur Folge, daß ein mit einem zweipoligen Magnetkörper versehener Bewegungssensor, der im übrigen den Fig. 1 und 2 entspricht, nur eine vergleichsweise geringe Empfindlichkeit besitzt.

In der Fig. 4 sind die gleichen Verhältnisse wie in Fig. 3 für einen kreisscheibenförmigen Magnetkörper K4 dargestellt, der zwei einander diametral gegenüberliegende Nordpole und zwei einander diametral gegenüberliegende Südpole aufweist, wobei alle Magnetpole die gleiche Polstärke besitzen. Die Magnetpole sind auf einer Kreislinie jeweils im Abstand von 90° voneinander angeordnet. Wie im Falle der Fig. 3 ist für beliebige Stellungen des Magnetkörpers die Summe der Kräfte FN und FS gleich Null. Außerdem läßt sich leicht feststellen, daß im Gegensatz zu der Fig. 3 für beliebige Stellungen des Magnetkörpers K4 auch die Summe der durch die Kräfte FN und FS ausgeübten Drehmomente gleich Null ist. Es wird also deutlich, daß im Falle der Fig. 4 der Magnetkörper K4 sich hinsichtlich der durch das homogene Magnetfeld auf ihn ausgeübten Kräfte in einem indifferenten Gleichgewichtszustand befindet. Dabei ist leicht festzustellen, daß dies nicht nur, wie der Einfachheit halber in Fig. 4 dargestellt, für ein ebenes, sondern auch für ein räumliches homogenes Magnetfeld, und nicht nur für einen kreisscheibenförmigen, sondern auch einen kugelförmigen Magnetkörper gilt. Übertragen auf den erfindungsgemäßen Bewegungssensor gemäß den Fig. 1 und 2 bedeutet dies, daß ein homogenes magnetisches Störfeld keine Bewegungen des kugelförmigen Magnetkörpers 3 relativ zu der Spule 4 hervorrufen kann, so daß der erfindungsgemäße Bewegungssensor störunempfindlich ist. Außerdem kann ein homogenes Magnetfeld den vierpoligen Magnetkörper K4 infolge des Umstandes, daß sich dieser bezüglich der auf die Magnetpole ausgeübten Kräfte in einem indifferenten Gleichgewicht befindet, nicht dämpfen. Es wird also deutlich, daß ein mit einem vierpoligen Magnetkörper K4 versehener erfindungsgemäßer Bewegungssensor MS eine hohe Empfindlichkeit besitzt und bereits auf geringste Bewegungen anspricht.

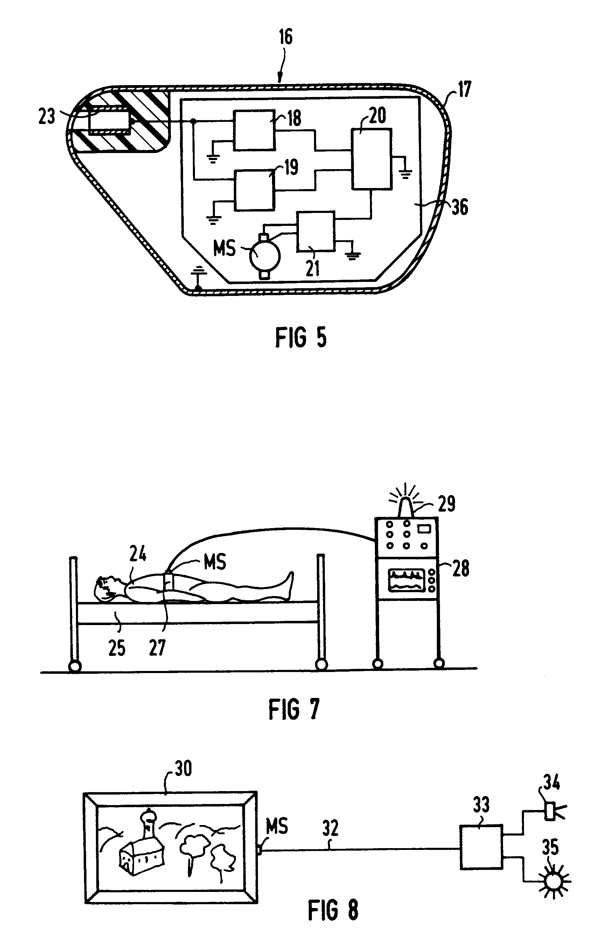

Die Fig. 5 verdeutlicht die Verwendung des Bewegungssensors MS nach den Fig. 1 und 2 in einem Herzschrittmacher 16. Der Herzschrittmacher 16, der zur Implantation in den Körper eines Patienten vorgesehen ist, besitzt ein aus einem elektrisch leitenden Werkstoff, z.B. Titan, gebildetes, hermetisch dichtes, flaches Gehäuse 17, das eine die Bauelemente des Herzschrittmachers 16 tragende gedruckte Schaltung 36 umschließt. Dabei liegt die gedruckte Schaltung 36 etwa parallel zu den Flachseiten des Gehäuses 17. Der Herzschrittmacher 16 weist einen Stimulationsimpuls-Generator 18 und eine Detektorschaltung 19 auf, die jeweils mit einer Steuerlogik 20 verbunden sind. Die Steuerlogik 20 veranlaßt den Stimulationsimpuls-Generator 18 immer dann zur Abgabe eines Stimulationsimpulses, wenn im Anschluß an einen mittels der Detektoreinrichtung 19 detektierten natürlichen Herzschlag oder einen von dem Stimulationsimpuls-Generator 18 abgegebenen Stimulationsimpuls nach Ablauf eines einer bestimmten Herzschlagfrequenz entsprechenden Zeitintervalls mittels der Detektoreinrichtung 19 kein natürlicher Herzschlag detektiert wurde. Dabei paßt die Steuerlogik 20 die Dauer des der Herzschlagfrequenz entsprechenden Zeitintervalls der jeweils vorhandenen körperlichen Aktivität des Patienten an. Hierzu ist die Steuerlogik 20 mit einer Signalaufbereitungsschaltung 21 verbunden, an die ein in Fig. 5 schematisch angedeuteter erfindungsgemäßer Bewegungssensor MS angeschlossen ist. Der Bewegungssensor MS ist in geeigneter Weise, z.B. durch Kleben oder formschlüssig an der gedruckten Schaltung 36 befestigt. Die Steuerlogik 20 stellt die Dauer des der Herzschlagfrequenz entsprechenden Zeitintervalls in Abhängigkeit von dem ihr von der Signalaufbereitungsschaltung 21 zugeführten Signal zwischen einem unteren Grenzwert, der einer sehr geringen bzw. fehlenden körperlichen Aktivität des Patienten entspricht (z.B. 60 Schläge pro Minute) und einem oberen Grenzwert, der einer sehr hohen körperlichen Aktivität des Patienten entspricht (z.B. 150 Schläge pro Minute), ein. Der Bewegungssensor MS ist derart an der gedruckten Schaltung 36 befestigt, daß die Rotationsachse seines Hohlraumes 1 im wesentlichen rechtwinklig zur Ebene der gedruckten Schaltung 36 verläuft. Außerdem ist im Falle des Bewegungssensors MS gemäß den Fig. 1 und 2 vorgesehen, daß der Durchmesser des kugelförmigen Magnetkörpers 3 wenigstens gleich dem minimalen Krümmungsradius des den Hohlraum 1 bildenden Rotationsellipsoids ist. Da der Herzschrittmacher 16 gewöhnlich derart im Brustbereich des Patienten implantiert wird, daß die Flachseiten des Gehäuses 17 parallel zur Körpervorderseite des Patienten verlaufen, verläuft die Rotationsachse des Hohlraumes 1 bei flachliegendem Patienten parallel zur Richtung der Schwerkraft. Bei flachliegendem Patienten stellt sich dann der in Fig. 6 dargestellte Betriebszustand des erfindungsgemäßen Bewegungssensors MS ein, in dem der kugelförmige Magnetkörper 3 unter der Wirkung der Schwerkraft, die in Fig. 6 durch einen mit G bezeichneten Pfeil angedeutet ist, längs einer Kreislinie an der Mantelfläche des Rotationsellipsoid förmigen Hohlraumes 1 anliegt. Der Magnetkörper 3 nimmt dann also unter der Wirkung der Schwerkraft eine "Arretierlage" ein, in der ein Abrollen auf der Wandung des Hohlraumes 1 und damit Bewegungen relativ zu der Spule 4 ausgeschlossen sind. Durch diese Maßnahme sind selbst unter ungewöhnlichen Schlafbedingungen, z.B. im Schlafwagen eines Zuges, physiologisch unbegründete Steigerungen der Stimulationsfrequenz ausgeschlossen, da der Bewegungssensor MS sozusagen die Empfindlichkeit Null besitzt, solange sich der Magnetkörper 3 in der "Arretierlage" befindet. Erst wenn der Patient wieder aufsteht, ist der Magnetkörper 3 wieder in der Lage, auf der Wandung des Hohlraumes 1 frei abzurollen, so daß eine der körperlichen Aktivität des Patienten angepaßte Steuerung der Stimulationsfrequenz erfolgen kann. Dabei kann vorgesehen sein, daß die Signalaufbereitungsschaltung 21 mit der Steuerlogik 20 derart zusammenwirkt, daß dann, wenn über einen längeren Zeitraum hinweg kein auf eine körperliche Aktivität des Patienten hindeutendes Signal aufgetreten ist, sich der Magnetkörper 3 also in einer "Arretierlage" befand, bei Auftreten eines auf eine körperliche Aktivität des Patienten hindeutenden Signales für einen kurzen Zeitraum eine besonders hohe Stimulationsfrequenz eingestellt wird, um den Patienten bei der Aufnahme seiner körperlichen Aktivität zu unterstützen. Der Durchmesser des kugelförmigen Magnetkörpers 3 muß nicht notwendigerweise größer als der minimale Krümmungsradius des Hohlraumes 1 sein, was die Voraussetzung dafür ist, ein Abrollen des Magnetkörpers 3 auf der Wandung des Hohlraumes 1 völlig zu unterbinden. In bestimmten Fällen kann es vielmehr auch ausreichen, wenn der Durchmesser des Magnetkörpers 3 den minimalen Krümmungsradius des Hohlraumes 1 annähernd erreicht. In diesem Falle ist bei parallel zur Richtung der Schwerkraft ausgerichteter Rotationsachse des Hohlraumes 1 ein freies Abrollen des Magnetkörpers 3 auf der Wandung des Hohlraumes 1 zwar nicht völlig unterbunden, jedoch stark erschwert, was zu einer stark herabgesetzten Empfindlichkeit des Bewegungssensors führt.

Der Stimulationsimpuls-Generator 18 besitzt übrigens einen das Stimulationspotential führenden Ausgang und einen ein Bezugspotential führenden Ausgang, wobei letzterer durch ein Massesymbol gekennzeichnet ist. In entsprechender Weise besitzt die Detektoreinrichtung 19 einen Eingang, an dem das der elektrischen Aktivität des zu stimulierenden Herzens entsprechende Potential liegt, und einen Anschluß, der das Bezugspotential führt, wobei letzterer wieder durch das Massesymbol gekennzeichnet ist. Sowohl der das Stimulationspotential führende Ausgang des Stimulationsimpuls-Generators 18 als auch der Eingang der Detektoreinrichtung 19 sind mit einer Anschlußbuchse 23 verbunden, an die eine nicht dargestellte unipolare, endokardielle Elektrode angeschlossen werden kann, die durch das Venensystem des Patienten zu dessen Herz geführt ist. Die Elektrode dient also einerseits dazu, das Herz des Patienten mit den mittels des Stimulationsimpuls-Generators 18 erzeugten Stimulationsimpulsen zu beaufschlagen, und andererseits dazu, der Detektoreinrichtung 19 das der elektrischen Aktivität des Herzens entsprechende Signal zuzuführen. Das Bezugspotential wird über das Gehäuse 17 des Herzschrittmachers 16 an das den Herzschrittmacher 16 umgebende Gewebe des Patienten angelegt, was dadurch angedeutet ist, daß das Gehäuse 17 mit einem Massesymbol versehen ist.

Infolge des Umstandes, daß der erfindungsgemäße Bewegungssensor 22 weder durch das Magnetfeld der Erde noch durch von elektrischen Geräten ausgehende niederfrequente magnetische Felder gestört werden kann, werden auch geringe körperliche Aktivitäten sicher detektiert, so daß der mit dem erfindungsgemäßen Bewegungssensor MS versehene Herzschrittmacher 16 insbesondere für ältere Patienten geeignet ist. Als weiterer Vorteil kommt hinzu, daß bei liegendem Patienten ein physiologisch unbegründeter Anstieg der Stimulationsfrequenz praktisch ausgeschlossen ist, da bei dieser Patientenlage ein Abrollen des kugelförmigen Magnetkörpers 3 auf der Wandung des Hohlraumes 1 unmöglich ist.

Aus den gleichen Gründen, die den erfindungsgemäßen Bewegungssensor zur Verwendung in Herzschrittmachern prädestinieren, ist dieser auch zur Überwachung von bettlägerigen Patienten besonders geeignet. Dies ist in Fig. 7 am Beispiel eines der Intensivpflege bedürftigen, z.B. narkotisierten oder im Koma liegenden Patienten 24 verdeutlicht. Dieser liegt in einem Bett 25 und ist mit einem einen erfindungsgemäßen Bewegungssensor MS enthaltenenden Kästchen versehen, das mittels eines Gurtes 27 an seinem Brustkorb befestigt ist. Der in dem Kästchen enthaltene Bewegungssensor MS steht über eine Leitung mit einer Überwachungseinrichtung 28 in Verbindung, die ein Lichtsignal 29 aufweist, das aktiviert wird, sobald die körperliche Aktivität des Patienten 24 ein bestimmtes einstellbares Niveau übersteigt. Die Überwachungseinrichtung 28 dient außerdem zur Überwachung weiterer physiologischer Funktionen des Patienten, wie z.B. dessen Herz- und Atemtätigkeit.

Die Verwendung des erfindungsgemäßen Bewegungssensors MS als Diebstahlsensor ist in Fig. 8 verdeutlicht. Hier ist schematisch ein Gemälde 30 angedeutet, an dessen Rahmen ein erfindungsgemäßer Bewegungssensor MS angebracht ist, der über eine Leitung mit einer Detektorschaltung 33 verbunden ist. Sobald das Gemälde 30 bewegt bzw. die Leitung 32 durchtrennt wird, aktiviert die Detektorschaltung eine akustische Signaleinrichtung 34 und/oder eine optische Signaleinrichtung 35. Infolge der beschriebenen Eigenschaften des erfindungsgemäßen Bewegungssensors MS kann dieser im Gegensatz zu bekannten induktiven Bewegungssensoren nicht ohne weiteres mittels eines Permanentmagneten von einem Dieb außer Funktion gesetzt werden.

In der Regel wird es sowohl bei zur Überwachung bettlägriger Patienten als auch bei zur Diebstahlsicherung vorgesehenen Bewegungssensoren MS angebracht sein, deren Hohlraum 1 kugelförmig auszubilden, so daß für beliebige Lagen des zu überwachenden Objektes eine gleich hohe Empfindlichkeit des Bewegungssensors MS gewährleistet ist. Auch bei zur Verwendung in Herzschrittmachern vorgesehenen Bewegungssensoren MS kann es zweckmäßig sein, den Hohlraum 1 kugelförmig auszubilden, z.B. dann, wenn mittels zusätzlicher Sensoren festgestellt werden kann, ob der Patient sich im Zustand der Ruhe, z.B. in einer liegenden Position, befindet.

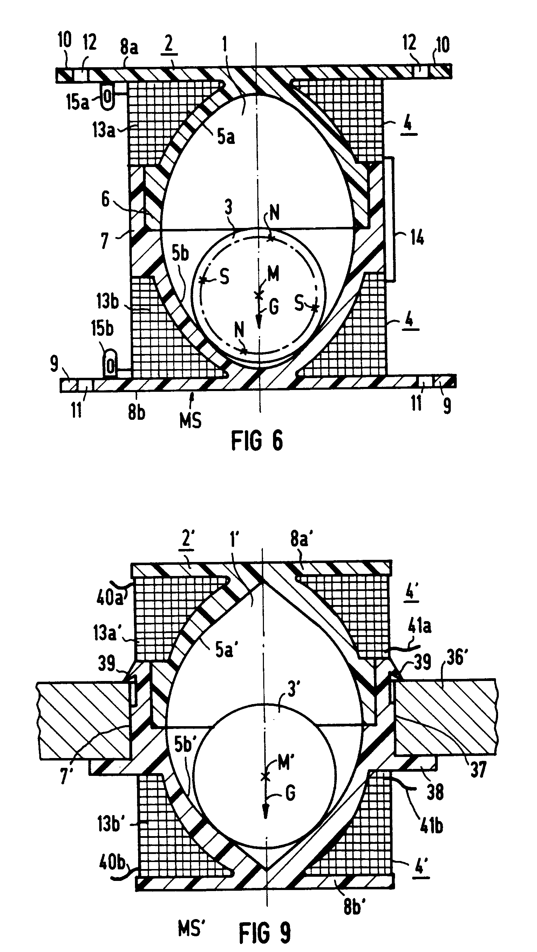

Ein weiteres Ausführungsbeispiel eines insbesondere zur Verwendung in einem Herzschrittmacher vorgesehenen erfindungsgemäßen Bewegungssensors ist in der Fig. 9 dargestellt. Dieses unterscheidet sich von dem zuvor beschriebenen Ausführungsbeispiel nur unwesentlich, so daß für jeweils gleiche Teile die gleichen Bezugszeichen verwendet werden, die im Falle der Fig. 9 jeweils mit einem ′ versehen sind. Ein erster Unterschied des Bewegungssensors MS′ gegenüber dem zuvor beschriebenen besteht darin, daß das Gehäuse 2′ einen zu der in Fig. 9 strichpunktiert eingetragenen Rotationsachse rotationssymmetrischen Hohlraum 1′ etwa zitronenförmiger Gestalt umschließt. Auch im Falle des Bewegungssensors MS nimmt also der Magnetkörper 3′, der vorzugsweise dem gemäß dem zuvor beschriebenen Ausführungsbeispiel entspricht, eine "Arretierlage" ein, in der ein Abrollen auf der Wandung des Hohlraumes 1′ ausgeschlossen ist, wenn die Rotationsachse des Hohlraumes 1' im wesentlichen parallel zur Richtung der Schwerkraft verläuft. Dabei bietet die zitronenförmige Gestalt des Hohlraumes 1′ den Vorteil, daß der Magnetkörper 3′ unabhängig von der Größe seines Durchmessers bei der beschriebenen Ausrichtung des Bewegungssensors M′ relativ zur Richtung der Schwerkraft vollständig am Abrollen auf der Wandung des Hohlraumes 1′ gehindert ist.

Ein weiterer Unterschied des Bewegungssensors MS′ gegenüber dem zuvor beschriebenen besteht in einer etwas andersartigen Gestaltung des Gehäuses 2′. Dieses ist dazu vorgesehen, in die Bohrung 37 einer gedruckten Schaltung 36′ montiert zu werden. Die Flansche 8a′ und 8b′ der Gehäuseteile 5a′ und 5b′ sind daher kreisscheibenförmig ohne irgendwelche Ansätze ausgeführt. Stattdessen ist das Gehäuseteil 5b′ im Bereich seines dem Flansch 8b′ benachbarten Endes mit einem Kragen 38 versehen, der bei montiertem Bewegungssensor MS′ an der einen Seite der gedruckten Schaltung 36′ abliegt. Im Bereich seines freien Endes ist der Zentrierrand 7′ mit einer Anzahl von elastisch nachgiebigen Klauen 39 versehen, die sich beim Einführen des Bewegungssensors MS′ in die Bohrung 37 radial nach innen verformen, bei vollständig in die Bohrung 37 eingeführtem Bewegungssensor MS′ wieder radial nach außen federn und so eine spielfreie Halterung des Bewegungssensors MS′ in der Bohrung 37 bewirken. Unter Bezugnahme auf Fig. 5 wird deutlich, daß durch diese Maßnahmen gewährleistet ist, daß der Einbau des Bewegungssensors MS′ in einen Herzschrittmacher in der Weise erfolgt, daß sich der Magnetkörper 3′ bei flachliegendem Patienten in einer "Arretierlage" befindet.

Die Enden 40a, 41a bzw. 40b, 41b der Spulenabschnitte 13a′ und 13b′ der Spule 4′ werden in nicht gezeigter Weise unmittelbar mit den hierfür vorgesehenen Lötpunkten der gedruckten Schaltung 36′ verlötet.

Im Falle des Ausführungsbeispieles nach den Fig. 1, 2 und 6, beziehungsweise des nach der Fig. 9, besitzt der Magnetkörper 3 je zwei Süd- und Nordpole. Im Rahmen der Erfindung kann der Magnetkörper 3 jedoch auch eine größere Anzahl von Magnetpolen aufweisen, sofern nur gewährleistet ist, daß die Anzahl der Südpole gleich der der Nordpole ist. Außerdem ist im Falle des beschriebenen Ausführungsbeispieles vorgesehen, daß sämtliche Magnetpole die gleiche Polstärke besitzen, auf einer Kreislinie angeordnet sind und zueinander jeweils den gleichen Winkelabstand aufweisen. Im Rahmen der Erfindung kann jedoch auch eine andere Anordnung getroffen sein, sofern nur sichergestellt ist, daß die Polstärke der Magnetpole so bemessen ist und die Magnetpole an dem Magnetkörper so plaziert sind, daß sich dieser in einem homogenen Magnetfeld in einer indifferenten Gleichgewichtslage befindet.

Vorzugsweise besteht der Magnetkörper aus einem magnetisierbaren Werkstoff, z.B. einer Eisen-Legierung, und ist in der erforderlichen Weise magnetisiert. Der Magnetkörper kann aber auch aus einem nichtmagnetisierbaren Werkstoff, z.B. einem polymeren Werkstoff, bestehen, in den in geeigneter Weise Magnete eingesetzt sind.

## Patentansprüche

1. Mit einem Objekt verbindbarer induktiver Bewegungssensor (MS; MS'), welcher eine Spule (4, 4') und einen relativ zu der Spule (4; 4') beweglichen Magnetkörper (3; 3') aufweist, **dadurch gekennzeichnet,** daß der Magnetkörper (3; 3') wenigstens vier Magnetpole aufweist, wobei die Anzahl der Südpole (S) gleich der der Nordpole (N) ist, und daß die Polstärke der Magnetpole so bemessen ist und die Magnetpole an dem Magnetkörper (3; 3') so plaziert sind, daß sich der Magnetkörper (3; 3') in einem homogenen Magnetfeld bezüglich der auf die Magnetpole ausgeübten Kräfte in einer indifferenten Gleichgewichtslage befindet.

2. Bewegungssensor nach Anspruch 1, **dadurch gekennzeichnet,** daß der Bewegungssensor (MS; MS') ein einen Hohlraum (1; 1') umschließendes Gehäuse (2; 2') aufweist, wobei der Magnetkörper (3; 3') in dem Hohlraum (1; 1') aufgenommen ist, und daß das Gehäuse (2; 2') von der Spule (4; 4') umgeben ist.

3. Bewegungssensor nach Anspruch 2, **dadurch gekennzeichnet,** daß die Spule (4; 4') auf die Außenseite des Gehäuses (2; 2') gewickelt ist.

4. Bewegungssensor nach einem der Ansprüche 1 bis 3, **dadurch gekennzeichnet,** daß die Magnetpole jeweils die gleiche Polstärke besitzen und in gleichen Winkelabständen voneinander derart auf einer Kreislinie angeordnet sind, daß auf einen Nordpol (N) jeweils ein Südpol (S) folgt.

5. Bewegungssensor nach einem der Ansprüche 1 bis 4, **dadurch gekennzeichnet,** daß der Magnetkörper (3; 3') kugelförmig ausgebildet ist.

6. Bewegungssensor mit den Merkmalen wenigstens der Ansprüche 2 und 5, **dadurch gekennzeichnet,** daß der kugelförmige Magnetkörper (3; 3') auf der Wandung des Hohlraumes (1; 1') abrollt.

7. Bewegungssensor nach einem der Ansprüche 2 bis 6, **dadurch gekennzeichnet,** daß der Hohlraum (1) rotationssymmetrisch ausgebildet ist.

8. Bewegungssensor nach Anspruch 7 oder 8, **dadurch gekennzeichnet,** daß der Hohlraum (1; 1') derart geformt ist, daß bei wenigstens einer Ausrichtung des Bewegungssensors (MS; MS') relativ zur Richtung der Schwerkraft ein Abrollen des Magnetkörpers (3; 3') auf der Wandung des Hohlraumes (1; 1') zumindest erschwert ist.

9. Bewegungssensor nach Anspruch 7 oder 8, **dadurch gekennzeichnet,** daß der Hohlraum (1') rotationssymmetrisch ausgebildet ist.

10. Bewegungssensor nach Anspruch 7, 8 oder 9, **dadurch gekennzeichnet,** daß der Hohlraum (1) wenigstens annähernd die Form eines Rotationsellipsoids aufweist.

11. Bewegungssensor nach Anspruch 7, 8 oder 9, **dadurch gekennzeichnet,** daß der Hohlraum (1') zitronenförmig ausgebildet ist.

12. Verwendung des Bewegungssensors nach einem der Ansprüche 1 bis 11 in einem Herzschrittmacher (16), dessen Stimulationsfrequenz in Abhängigkeit von der körperlichen Aktivität des den Herzschrittmacher (16) tragenden Lebewesens gesteuert wird.

13. Verwendung des Bewegungssensors nach einem der Ansprüche 1 bis 11 zur Überwachung eines bettlägerigen Patienten (24).

14. Verwendung des Bewegungssensors nach einem der Ansprüche 1 bis 11 als Diebstahlsensor.

## Claims

1. Inductive movement sensor (MS; MS'), which can be connected to an object and has a coil (4; 4') and a magnet body (3; 3') movable relative to the coil (4; 4'), characterised in that the magnet body (3; 3') has at least four magnet poles, the number of the south poles (S) being equal to that of the north poles (N), and in that the pole strength of the magnet poles is dimensioned such that, and the magnet poles are placed on the magnet body (3; 3') such that the magnet body (3; 3') is located in a homogeneous magnetic field in a position of neutral equilibrium relative to the forces exerted on the magnet poles.

2. Movement sensor according to Claim 1, characterised in that the movement sensor (MS; MS') has a housing (2; 2') enclosing a cavity (1; 1'), the magnet body (3; 3') being accommodated in the cavity (1; 1'), and in that the housing (2; 2') is surrounded by the coil (4; 4').

3. Movement sensor according to Claim 2, characterised in that the coil (4; 4') is wound onto the outside of the housing (2; 2').

4. Movement sensor according to one of Claims 1 to 3, characterised in that the magnet poles have the same pole strength in each case and are arranged at the same angular distance from one another on a circular line in such a way that a south pole (S) follows a north pole (N) in each case.

5. Movement sensor according to one of Claims 1 to 4, characterised in that the magnet body (3; 3') is constructed in the shape of a sphere.

6. Movement sensor having the features of at least Claims 2 and 5, characterised in that the spherically shaped magnet body (3; 3') rolls on the wall of the cavity (1; 1').

7. Movement sensor according to one of Claims 2 to 6, characterised in that the cavity (1) is constructed in a rotationally symmetrical fashion.

8. Movement sensor according to Claim 7 or 8, characterised in that the cavity (1; 1') is formed in such a way that in at least one alignment of the movement sensor (MS; MS') relative to the direction of gravity rolling of the magnet body (3; 3') on the wall of the cavity (1; 1') is at least rendered difficult.

9. Movement sensor according to Claim 7 or 8, characterised in that the cavity (1') is constructed in a rotationally symmetrical fashion.

10. Movement sensor according to Claim 7, 8 or 9, characterised in that the cavity (1) has at least approximately the shape of an ellipsoid of revolution.

11. Movement sensor according to Claim 7, 8 or 9, characterised in that the cavity (1') is constructed in the shape of a lemon.

12. Use of the movement sensor according to one of Claims 1 to 11 in a pacemaker (16) whose stimulation frequency is controlled as a function of the bodily activity of the patient carrying the pacemaker (16).

13. Use of the movement sensor according to one of Claims 1 to 11 for monitoring a bedridden patient (24).

14. Use of the movement sensor according to one of Claims 1 to 11 as a theft sensor.

## Revendications

1. Capteur inductif de déplacement (MS; MS') pouvant être relié à un objet et qui possède une bobine (4,4') et un corps magnétique (3;3') déplaçable par rapport à la bobine (4;4'), caractérisé par le fait que le corps magnétique (3;3') possède au moins quatre pôles magnétiques, le nombre des pôles sud (S) étant égal au nombre des pôles nord (N), et que l'épaisseur des pôles magnétiques est dimensionnée et les pôles magnétiques sont placés sur le corps magnétique (3;3') de telle sorte que le corps magnétique (3;3') est situé dans un champ magnétique homogène, dans une position d'équilibre indifférent par rapport aux forces appliquées aux pôles magnétiques.

2. Capteur de déplacement suivant la revendication 1, caractérisé par le fait que le capteur de déplacement (MS; MS') comporte un boîtier (2;2'), qui entoure une cavité (1; 1'), le corps magnétique (3;3') étant logé dans la cavité (1; 1'), et que le boîtier (2;2') est entouré par la bobine (4;4').

3. Capteur de déplacement suivant la revendication 2, caractérisé par le fait que la bobine (4;4') est enroulée sur le côté extérieur du boîtier (2;2').

4. Capteur de déplacement suivant l'une des revendications 1 à 3, caractérisé par le fait que les pôles magnétiques possèdent respectivement la même épaisseur et sont séparés par des distances angulaires identiques sur une ligne circulaire de sorte qu'un pôle sud (S) succède respectivement à un pôle nord (N).

5. Capteur de déplacement suivant l'une des revendications 1 à 4, caractérisé par le fait que le corps magnétique (3;3') possède une forme sphérique.

6. Capteur de déplacement comportant les caractéristiques suivant les revendications 2 et 5, caractérisé par le fait que le corps magnétique de forme sphérique (3;3') roule sur la paroi de la cavité (1;1').

7. Capteur de déplacement suivant l'une des revendications 2 à 6, caractérisé par le fait que la cavité (1) présente une symétrie de révolution.

8. Capteur de déplacement suivant la revendication 7 ou 8, caractérisé par le fait que la cavité (1;1') est conformée de telle sorte que pour au moins un alignement du capteur de déplacement (MS;MS') par rapport à la direction de la force de pesanteur, un roulement du corps magnétique (3;3') contre la paroi de la cavité (1;1') est pour le moins rendu compliqué.

9. Capteur de déplacement suivant la revendication 7 ou 8, caractérisé par le fait que la cavité (1') est réalisée avec une symétrie de révolution.

10. Capteur de déplacement suivant la revendication 7, 8 ou 9, caractérisé par le fait que la cavité (1) possède au moins approximativement la forme d'un ellipsoide de révolution.

11. Capteur de déplacement suivant la revendication 7, 8 ou 9, caractérisé par le fait que la cavité (1') est réalisée avec une forme de citron.

12. Utilisation du capteur de déplacement suivant l'une des revendications 1 à 11 dans un stimulateur cardiaque (16), dont la fréquence de stimulation est commandée en fonction de l'activité corporelle de l'être vivant portant le stimulateur cardiaque (16).

13. Utilisation du capteur de déplacement suivant l'une des revendications 1 à 11 pour le contrôle d'un patient grabataire (24).

14. Utilisation du capteur de déplacement suivant l'une des revendications 1 à 11 en tant que détecteur d'effraction.
